# EUROPEAN PATENT SPECIFICATION

(11) **EP 3 705 098 B1**
(45) Date of publication and mention of the grant of the patent: **15.03.2023**
(21) Application number: 18894919.2
(22) Date of filing: 17.12.2018
(51) Int. Cl.: A61F 13/49, A61F 13/15, A61F 13/539

(54) **ABSORBENT ARTICLE**
ABSORBIERENDER ARTIKEL
ARTICLE ABSORBANT

(30) Priority: 28.12.2017 JP 2017254069
(43) Date of publication of application: 09.09.2020
(73) Proprietor: Unicharm Corporation, Shikokuchuo-shi, Ehime 799-0111 (JP)
(72) Inventor: KUDO, Etsuko, Kanonji-shi, Kagawa 769-1602 (JP)
(74) Representative: Dolleymores
(86) International application number: PCT/JP2018/046295
(87) International publication number: WO 2019/131267

(56) References cited:
- EP-A1- 2 177 190
- JP-A- 2009 039 441
- JP-A- 2009 539 467
- JP-A- 2015 171 502
- JP-A- 2017 012 403
- JP-A- 2017 012 403
- US-A1- 2017 105 881

## Description

### [TECHNICAL FIELD]

The present invention relates to an absorbent article such as a disposable diaper.

### [BACKGROUND ART]

As described in Patent Literature 1, an absorbent article such as a so-called underpants type disposable diaper is known. The absorbent article described in Patent Literature 1 has a waistline region arranged around the waistline of the wearer and a crotch region arranged in the crotch region of the wearer. The waistline region includes a front waistline region and a rear waistline region, and the front waistline region and the rear waistline region are engaged together by an engaging portion. An absorbent core absorbing body fluid of the wearer is provided in the crotch region.

### [CITATION LIST]

### [PATENT LITERATURE]

[Patent Literature 1] JP 2017-140072 A

US 2017/0105881 A1, JP 2017 012403 A and EP 2177190 A1 also disclose so-called underpants type disposable diapers.

### [SUMMARY OF INVENTION]

The inventor of the present application has found the following problem as a result of intensive research. That is, an assistant such as a parent for an infant or toddler exchanges absorbent articles for a wearer such as an infant or toddler. When removing the absorbent article from the laid wearer, the assistant may tear a part of the waistline region of the absorbent article and wipe the wearer's skin with the torn waistline region.

However, if the waistline region is too hard, it may be difficult to wipe off loose stool or may damage the wearer's skin in the case where the loose stool enters a recess such as a region around the wearer's genitals or a region around the hole through which the wearer's leg passes. If the waistline region is too soft, on the other hand, when grasping the waistline region of the absorbent article to pull up the absorbent article to the waistline region of the wearer at the time of mounting, the pulling force may not be sufficiently transmitted from the waistline region to the absorbent core. In this case, it becomes difficult to pull up the absorbent core beyond the swelling of the wearer's skin, for example, the swelling of the buttocks, and it becomes difficult to sufficiently pull up the crotch region of the absorbent article.

Accordingly, there is a desire for an absorbent article easy to wear when putting on and easy to wipe the wearer's skin when removing.

The present invention provides the absorbent article of independent claim 1. The dependent claims specify preferred but optional features.

An absorbent article according to an embodiment includes a front-rear direction; a width direction orthogonal to the front-rear direction;
a front waistline region; a rear waistline region; a crotch region arranged between the front waistline region and the rear waistline region; a side engaging portion engaging the front waistline region and the rear waistline region; and an absorbent core provided at least in the crotch region. A folding line for folding the absorbent article in half extends along the width direction in the crotch region. A first distance from a front end edge of the absorbent core to a front end edge of the front waistline region is longer than a second distance from a rear end edge of the absorbent core to a rear end edge of the rear waistline region. A difference between a distance from the folding line to a rear end edge of the absorbent core and a distance from the folding line to a front end edge of the absorbent core is longer than a length in the front-rear direction of a region of the front waistline region overlapping the absorbent core. A difference between a distance from the folding line to a rear end edge of the absorbent core and a distance from the folding line to a front end edge of the absorbent core is longer than the second distance.

### [BRIEF DESCRIPTION OF DRAWINGS]

Fig. 1 is a schematic view of an absorbent article according to an embodiment.
Fig. 2 is a developed plan view of an absorbent article according to an embodiment.
Fig. 3 is a cross-sectional view of the absorbent article taken along a line A-A of Fig. 1.
Fig. 4 is a schematic side view of an absorbent article folded in half.

### [DESCRIPTION OF EMBODIMENTS]

### (1) Outline of Embodiment

According to the present specification and the accompanying drawings, at least the following matters are disclosed.

An absorbent article according to an embodiment includes a front-rear direction; a width direction orthogonal to the front-rear direction;
a front waistline region; a rear waistline region; a crotch region arranged between the front waistline region and the rear waistline region; a side engaging portion engaging the front waistline region and the rear waistline region; and an absorbent core provided at least in the crotch region. A folding line for folding the absorbent article in half extends along the width direction in the crotch region. A first distance from a front end edge of the absorbent core to a front end edge of the front waistline region is longer than a second distance from a rear end edge of the absorbent core to a rear end edge of the rear waistline region. A difference between a distance from the folding line to a rear end edge of the absorbent core and a distance from the folding line to a front end edge of the absorbent core is longer than a length in the front-rear direction of a region of the front waistline region overlapping the absorbent core.

According to the present aspect, a first distance from a front end edge of the absorbent core to the front end edge of the front waistline region is longer than a second distance from a rear end edge of the absorbent core to the rear end edge of the rear waistline region, and hence a region of the front waistline region in which the absorbent core does not exist is widened. Due to this, a soft region in the front waistline region is widened, and it thus becomes easy for the assistant to wipe the wearer's skin by the front waistline region without damaging the wearer's skin when removing the absorbent article.

On the other hand, the difference between the distance from the folding line to the rear end edge of the absorbent core and the distance from the folding line to the front end edge of the absorbent core is longer than the length in the front-rear direction of the region in the front waistline region overlapping the absorbent core. Therefore, in a state where the absorbent article is folded at the folding line, the absorbent core extends, in the rear waistline region, relatively long upward (dorsal side) relative to the front waistline region. Since the relatively high rigidity absorbent core extends long rearward the rear waistline region, when grasping the vicinity of the side engaging portion to pull up the absorbent article to the wearer's waistline, the pulling force is sufficiently transmitted to the absorbent core arranged in the rear waistline region. This makes it easy to pull up the absorbent core beyond the swelling of the wearer's buttocks.

As described above, it is possible to provide an absorbent article easy to wear when putting on and easy to wipe the wearer's skin when removing.

According to a preferred embodiment, the absorbent article includes an absorbent body having the absorbent core and a liquid-impermeable film provided on a non-skin surface side of the absorbent core. A region on a front side relative to a front end edge of the liquid-impermeable film accounts for 50% or more of a length of the side engaging portion in the front-rear direction.

According to the present aspect, the region without a liquid-impermeable film more solid than a sheet such as a nonwoven fabric accounts for 50% or more of the length of the side engaging portion, and it is hence possible to secure a wide region suitable for wiping the wearer's skin.

According to a preferred embodiment, an entire front region of the front waistline region on a front side relative to a front end edge of the absorbent core is constituted by four or more sheets.

The entire front region of the front waistline region on the front side relative to the front end edge of the absorbent core corresponds to a region where the wearer's skin is easily wiped in a state where the rear waistline region is laid under the wearer's buttocks and the side engaging portion is torn. The entire region where the wearer's skin is easily wiped is constituted of four or more sheets, and the body fluid hardly oozes, and hence the assistant's hands hardly get dirty.

According to a preferred embodiment, a number of sheets constituting an entire front region of the front waistline region on a front side relative to a front end edge of the absorbent core is equal to or less than a number of sheets constituting a rear region of the rear waistline region overlapping the front region in a state of being folded at the folding line.

According to the present aspect, it is possible to suppress the rigidity of the front region from becoming too high by not excessively increasing the number of sheets of the front region, and it is possible to easily wipe off the body fluid adhering to the skin by the front region. On the other hand, by not excessively reducing the number of sheets in the rear region, when grasping the vicinity of the side engaging portion to pull up the absorbent article to the wearer's waistline, the pulling force is sufficiently transmitted from the rear region to the absorbent core.

According to a preferred embodiment, a basis weight of a sheet constituting an entire front region of the front waistline region on a front side relative to a front end edge of the absorbent core is higher than a basis weight of a sheet constituting a rear region of the rear waistline region overlapping the front region in a state of being folded at the folding line.

According to the present aspect, by increasing the basis weight of the sheet in the front region, it is possible to sufficiently wipe off the body fluid adhering to the skin by the front region.

According to the present invention, a difference between a distance from the folding line to a rear end edge of the absorbent core and a distance from the folding line to a front end edge of the absorbent core is longer than the second distance.

The difference between the distance from the folding line to the rear end edge of the absorbent core and the distance from the folding line to the front end edge of the absorbent core is longer than the second distance from the rear end edge of the absorbent core to the rear end edge of the rear waistline region, and hence the relatively high rigidity absorbent core extends long rearward the rear waistline region. In this case, when grasping the vicinity of the side engaging portion to pull up the absorbent article to the wearer's waistline, the pulling force is sufficiently transmitted to the absorbent core arranged in the rear waistline region. This makes it easy to pull up the absorbent core beyond the swelling of the wearer's buttocks.

According to a preferred embodiment, an elastic thread-free region not having an elastic thread accounts for a continuous region of at least 30% of the entire front waistline region.

In the region having the elastic thread, there is a difference in contraction force between the part provided with the elastic thread and the part around it. The difference in the contraction force causes a large unevenness in the sheet between the elastic threads. In the present aspect, since the elastic thread-free region having no elastic thread accounts for a continuous region of at least 30% of the front waistline region, a large unevenness accompanying the contraction force of the elastic thread is not generated in a relatively wide range in the front waistline region. The region where such large unevenness is not generated is a region suitable for wiping the wearer's skin.

According to a preferred embodiment, the elastic thread-free region has a stretchable sheet stretchable in the width direction.

According to the present aspect, by providing the elastic thread-free region in the front waistline region with a stretchable sheet stretchable in the width direction, the front waistline region can be made stretchable in the width direction, whereby the front waistline region can be fitted to the wearer's skin.

Furthermore, the stretchable sheet tends to contract uniformly in the range where the stretchable sheet exists. Therefore, a large unevenness occurring with the contraction of the elastic thread is hardly generated. Accordingly, the elastic thread-free region in the front waistline region can be fitted to the wearer's skin by the stretchable sheet, and the elastic thread-free region can be maintained as a region suitable for wiping the wearer's skin.

According to a preferred embodiment, at least a part of the elastic thread-free region is located on a front side relative to a center of the side engaging portion in the front-rear direction.

According to the present aspect, the elastic thread-free region suitable for wiping the wearer's skin is located on the front side (ventral side). In the state where the rear waistline region is laid under the wearer's buttocks and the side engaging portion is torn, the operation range of the region on the front side (ventral side) of the front waistline region becomes relatively wide. Accordingly, it becomes easy for the assistant to wipe the wearer's skin using the elastic thread-free region in the state where the side engaging portion is torn.

According to a preferred embodiment, the elastic thread-free region extends rearward from a front end edge of the front waistline region.

In the state where the rear waistline region is laid under the wearer's buttocks and the side engaging portion is torn, the operation range of the region on the front side (ventral side) of the front waistline region becomes relatively wide. Accordingly, since the elastic thread-free region extends rearward from the front end edge of the front waistline region, the assistant can easily wipe the wearer's skin using the elastic thread-free region in the state where the side engaging portion is torn.

According to a preferred embodiment, at least a part of the elastic thread-free region is provided in a region where the absorbent core is extended in the front-rear direction.

The region where the absorbent core is extended in the front-rear direction is near the region where the wearer's skin gets dirty by the body fluid adhered to the absorbent core. Accordingly, by providing the elastic thread-free region suitable for wiping the wearer's skin in the region where the absorbent core is extended in the front-rear direction, it becomes easy to wipe the dirt adhered to the wearer's skin in the elastic thread-free region.

According to a preferred embodiment, at least a part of the elastic thread-free region is provided outside the absorbent core in the width direction.

The region outside the absorbent core in the width direction in the front waistline region is a region stain-resistant by the body fluid of the wearer. By providing the elastic thread-free region suitable for wiping the wearer's skin in such a stain-resistant region, it becomes possible to wipe the dirty adhered to the wearer's skin using a clean part of the front waistline region.

According to a preferred embodiment, the elastic thread-free region does not have a liquid-impermeable film.

According to the present aspect, since a liquid-impermeable film more solid than a sheet such as a nonwoven fabric is not provided in the elastic thread-free region, the elastic thread-free region can be made more suitable for wiping the wearer's skin.

According to a preferred embodiment, the elastic thread-free region has a liquid-impermeable film.

According to the present aspect, since the liquid-impermeable film is present in the elastic thread-free region suitable for wiping the skin, when the assistant wipes the body fluid adhering to the wearer's skin in the elastic thread-free region, the body fluid adhering to the elastic thread-free region can be suppressed from passing through, in the thickness direction, the sheet constituting the elastic thread-free region and adhering to the assistant's hands.

### (2) Structure of Absorbent Article

An absorbent article according to an embodiment will be described below by referring to the accompanying drawings. In the drawings, the same or similar parts are indicated by the same or similar reference signs. The drawings are illustrated schematically, and dimensional ratio and other variables differ from those of actual measurements. The actual measurements or the like, therefore, should be determined by referring to the following description. The drawings may include different relationships or ratios of measurements.

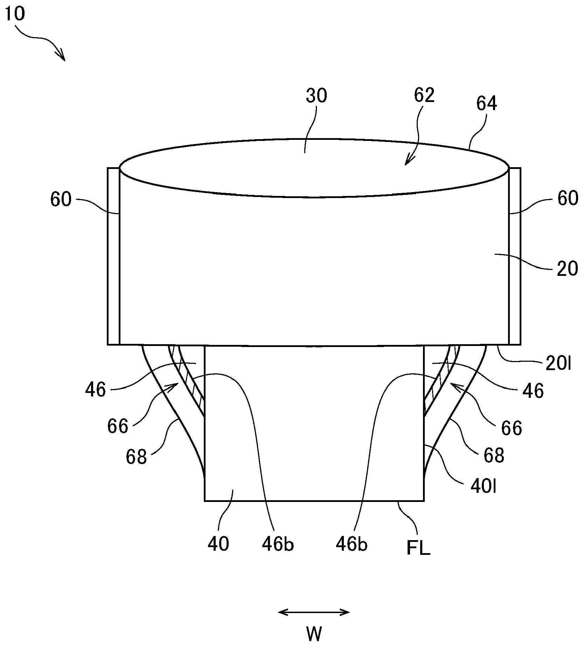

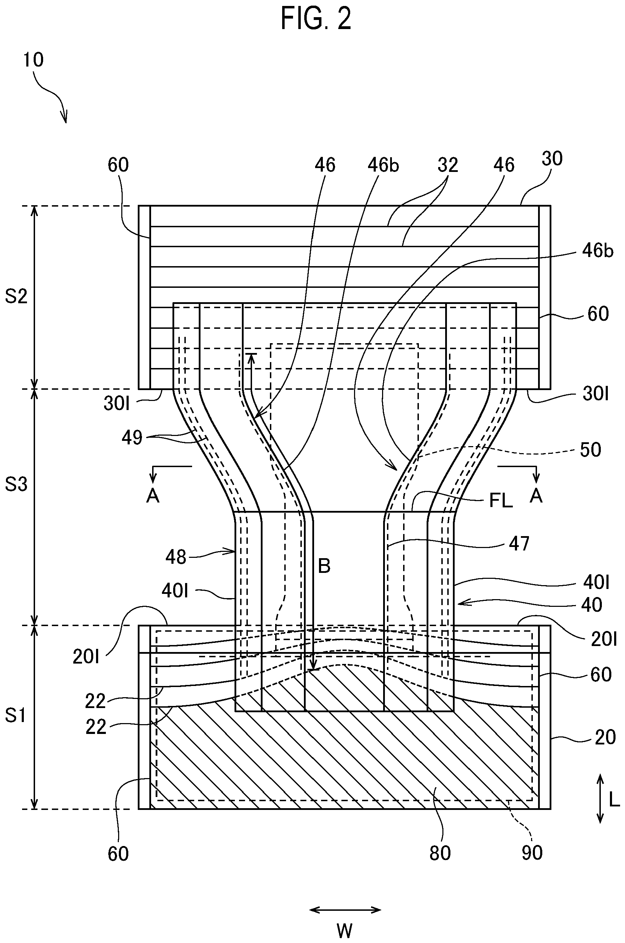

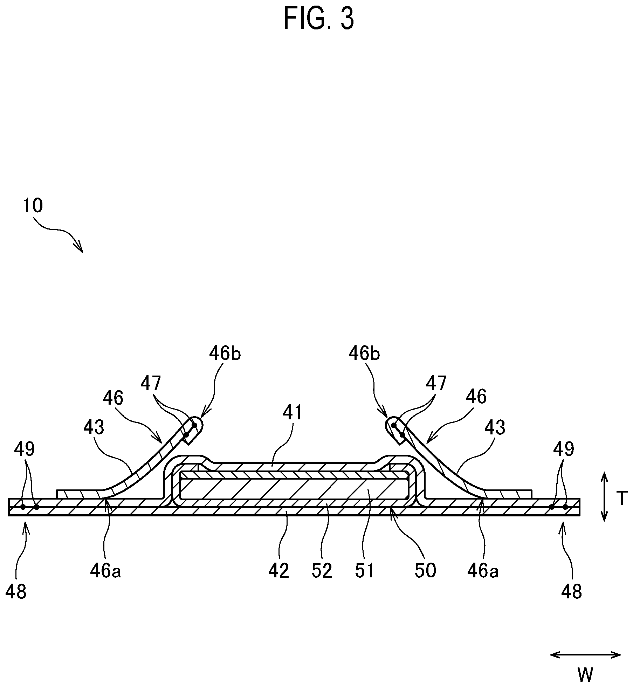

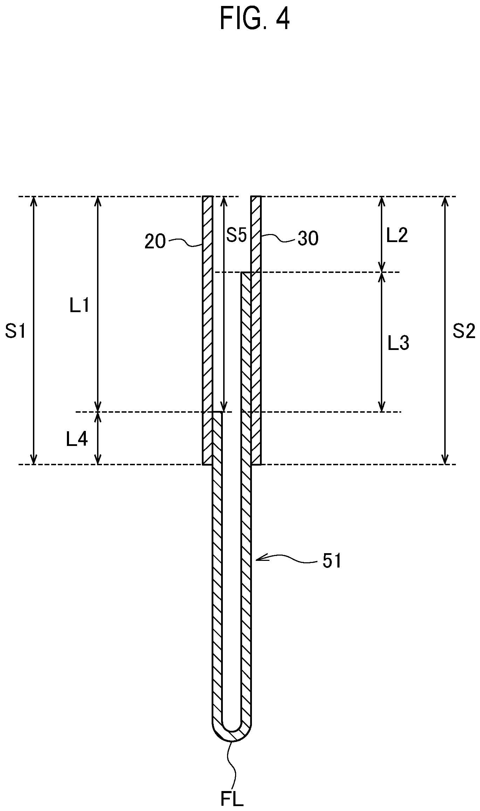

Fig. 1 is a schematic view of an absorbent article according to an embodiment. Fig. 2 is a developed plan view of an absorbent article according to an embodiment. Fig. 2 illustrates an extended state of the absorbent article extended till no wrinkles are formed in a state in which a side engaging portion described later is developed. Fig. 3 is a cross-sectional view of the absorbent article taken along a line A-A of Fig. 2. Fig. 4 is a schematic side view of an absorbent article folded in half. Note that FIG. 4 is a schematic diagram showing a relationship in length between an absorbent core 51 described later, a front waistline member 20 described later, and a rear waistline member 30 described later. In FIG. 4, configurations other than these members are not shown for simplification.

An absorbent article 10 includes a front-back direction L and a width direction W, both directions being orthogonal to each other in a developed state. The front-back direction L is defined by a direction extending to the front (ventral) side and the rear (dorsal) side of the body. In other words, the front-back direction L is a direction extending forward and backward in the developed absorbent article 10. Further, the absorbent article 10 includes a thickness direction T perpendicular to both the front-back direction L and the width direction W

The absorbent article 10 includes a front waistline region S1, a rear waistline region S2, and a crotch region S3. The front waistline region S1 is a region facing the front waistline of the wearer. The rear waistline region S2 is a region facing the rear waistline of the wearer. The crotch region S3 is a region located in the crotch of the wearer and disposed between the front waistline region S1 and the rear waistline region S2.

In the present embodiment, the absorbent article 10 may include a front waistline member 20, a rear waistline member 30, and an absorbent body 40. The front waistline member 20 is disposed in the front waistline region S1. The rear waistline member 30 is disposed in the rear waistline region S2. The front waistline member 20 and the rear waistline member 30 may be made of a sheet such as a nonwoven fabric.

A folding line FL which bisects the absorbent article 10 into front and rear portions may pass through the crotch region S3. The folding line FL extends in the width direction W. That is, in the state shown in Fig. 1, the absorbent article 10 is folded in half at the folding line FL extending along the width direction W as a base point.

A side engaging portion 60 may be provided at the end portion of the front waistline region S1 in the width direction W and the end portion of the rear waistline region S2 in the width direction W. The side engaging portion 60 is defined by a portion where the end portion of the front waistline region S1 in the width direction W and the end portion of the rear waistline region S2 in the width direction W are engaged with each other.

The end portion of the front waistline region S1 in the width direction W and the end portion of the rear waistline region S2 in the width direction W are locked by the side engaging portion 60. In this state, the absorbent article 10, a waistline opening 62 through which the torso is passed and a pair of leg openings 66 into which the legs of the wearer are inserted. A waistline opening edge 64 forming the waistline opening 62 may be defined by the front edge of the front waistline member 20 and the rear edge of the rear waistline member 30. Further, a leg opening edge 68 forming the leg opening 66 may be defined by a side edge 40l of the absorbent body 40 extending in the front-back direction L, a side 20l located behind the front waistline member 20, and a side 30l located in front of the rear waistline member 30.

Fig. 2 illustrates the state in which the side engaging portion 60 is released and the absorbent article 10 is developed. The side engaging portion 60 may extend in the front-back direction L on the front waistline member 20 and the rear waistline member 30. In this case, the boundary between the front waistline region S1 and the crotch region S3 may be defined by the rear edge of the side engaging portion 60 provided on the front waistline member 20. Similarly, the boundary between the rear waistline region S2 and the crotch region S3 may be defined by the front edge of the side engaging portion 60 provided on the rear waistline member 30.

One or more elastic members 32 that are stretchable in the width direction W may be provided in the rear waistline region S2. Alternatively, the elastic members 32 of the rear waistline region S2 may be made of a stretchable sheet that can expand and contract in the width direction W

One or more second elastic members 22 that are stretchable in the width direction W may be provided in the front waistline region S1. The second elastic member 22 is made of elastic threads. Further, a stretchable sheet 90 stretchable in the width direction W may be provided in the front waistline region S1. The stretchable sheet 90 may be provided in a region not overlapping the second elastic members 22 in the thickness direction T.

The absorbent body 40 is disposed across the front waistline member 20 and the rear waistline member 30. That is, the absorbent body 40 extends over the front waistline region S1, the rear waistline region S2, and the crotch region S3.

The absorbent body 40 may be formed separately from the front waistline member 20 and the rear waistline member 30. In this case, the absorbent body 40 may be bonded to the front waistline member 20 and the rear waistline member 30, respectively, in the front waistline region S1 and the rear waistline region S2.

The absorbent body 40 at least includes the absorber 50. The absorbent body 40 may also include a skin surface sheet 41 positioned on the skin surface side of the absorber 50 and a non-skin surface sheet 42 positioned on the non-skin surface side of the absorber 50. The skin surface sheet 41 may cover the absorber 50 on the skin surface side of the absorber 50. The non-skin surface sheet 42 may cover the absorber 50 on the non-skin surface side of the absorber 50.

The skin surface sheet 41 may be made of a liquid impermeable sheet, such as a nonwoven fabric or a perforated plastic film. The skin surface sheet 41 may be made of one sheet. Alternatively, the skin surface sheet 41 may be made of a stacked sheet in which a plurality of sheets are stacked.

The non-skin surface sheet 42 may include a liquid impermeable sheet. The liquid impermeable sheet may be made of a liquid impermeable film, such as a plastic film. The non-skin surface sheet42 may be made of one sheet. Alternatively, the non-skin surface sheet42 may be made of a laminated sheet in which a plurality of sheets is laminated. In this case, at least one of the plurality of sheets may be liquid impermeable.

The absorber 50 may include an absorbent core 51 and a core wrap 52 enclosing the absorbent core. The absorbent core 51 may include, for example, ground pulp, or superabsorbent polymer (SAP), or mixtures thereof. The core wrap 52 may be made of, for example, a tissue or a nonwoven fabric.

The absorbent core 51 is arranged at least in the crotch region S3. Preferably, the absorbent core 51 may extend from the front waistline region S1 to the rear waistline region S2 in the front-back direction L.

The absorbent core 51 may include a constricted region constricted inward in the width direction W at least in the crotch region S3. The narrowest portion of the absorbent core 51 in the width direction W may be provided in the crotch region S3. In this case, the folding line FL may pass through the constricted region of the absorbent core 51.

The absorbent body 40 may include a side sheet 43 covering both side portions of the skin surface sheet 41 in the width direction W. The side sheet 43 may be made of, for example, a sheet, such as a nonwoven fabric or a perforated plastic film.

The absorbent body 40 may include a pair of leakage preventing cuffs 46. The pair of leakage preventing cuffs 46 extends in the front-back direction L between the front waistline region S1 and the rear waistline region S2, and is disposed on both sides of the center of the absorbent article 10 in the width direction W. The pair of leakage preventing cuffs 46 is defined by a portion (a portion of range B in Fig. 2) that can rise toward the skin of the wearer.

The leakage preventing cuffs 46 may be made of the side sheet 43 and a third elastic member 47 provided on the side sheet 43. The third elastic member 47 is configured to be stretchable in the front-back direction. The third elastic member 47 may include one or more rubber threads. In a specific example, the inner side of the side sheet 43 in the width direction W is folded back to the outside, and the third elastic member 47 is disposed in the folded back portion of the side sheet 43. The inner edge of the side sheet 43 in the width direction W is not bonded to the skin surface sheet 41, and forms a free end that can rise from the skin surface sheet 41.

Each leakage preventing cuff 46 includes a rising fulcrum 46a and a rising apex 46b. The rising fulcrum 46a and the rising apex 46b extend in the front-back direction L from the front waistline region S1 to the rear waistline region S2. The rising fulcrum 46a is a non-rising point and corresponds to the root of the leakage preventing cuff 46. In the present embodiment, the rising fulcrum 46a corresponds to an edge line on the inner side in the width direction W of the bonding portion between the side sheet 43 and the skin surface sheet 41. The rising apex 46b corresponds to the apex of the leakage preventing cuff 46 when the leakage preventing cuff 46 rises. That is, the rising apex 46b is the free end of the leakage preventing cuff 46.

The absorbent body 40 may include a pair of gathers 48 in a region outside the pair of leakage preventing cuffs 46 in the width direction W. The pair of gathers 48 may extend in the front-back direction L from the front waistline region S1 to the rear waistline region S2.

The pair of gathers 48 may be made of a fourth elastic member 49 attached to a sheet that forms the absorbent body 40. The fourth elastic member 49 may be, for example, one or more rubber threads. Alternatively, the fourth elastic member 49 may be, for example, an elastic sheet having elasticity. The fourth elastic member 49 is bonded to a sheet that forms the absorbent body 40 in a manner stretching from its natural state. Accordingly, the fourth elastic member 49 can form gathers to contract the absorbent body 40 in the front-back direction L.

As shown in Fig. 4, a first distance L1 from the front end edge of the absorbent core 51 to the front end edge of the front waistline region S1 is longer than a second distance L2 from the rear end edge of the absorbent core 51 to the rear end edge of the rear waistline region S2. That is, the region where the absorbent core 51 does not exist is wide in the front waistline region S1. Due to this, the soft region in the front waistline region S1 is widened, and hence it becomes easy for the assistant to wipe the wearer's skin by the front waistline region S1 without damaging the wearer's skin when removing the absorbent article.

Furthermore, a difference L3 between the distance from the folding line FL to the rear end edge of the absorbent core 51 and the distance from the folding line FL to the front end edge of the absorbent core 51 is longer than a length L4 in the front-rear direction L of the region overlapping the absorbent core 51 of the front waistline region S1. Thus, in the state where the absorbent article 10 is folded at the folding line FL, the absorbent core 51 extends, in the rear waistline region S2, relatively long upward (dorsal side) relative to the front waistline region S1. Since the relatively high rigidity absorbent core 51 extends long rearward the rear waistline region S2, when grasping the vicinity of the side engaging portion 60 to pull up the absorbent article 10 to the wearer's waistline, the pulling force is sufficiently transmitted to the absorbent core 51 arranged in the rear waistline region S2. This makes it easier to pull up the absorbent core 51 beyond the swelling of the wearer's buttocks.

As described above, it is possible to provide the absorbent article 10 that allows the wearer to easily wear the absorbent article 10 when worn and to easily wipe the skin of the wearer when removing the absorbent article.

The difference L3 between the distance from the folding line FL to the rear end edge of the absorbent core 51 and the distance from the folding line FL to the front end edge of the absorbent core 51 is longer than the second distance L2 from the rear end edge of the absorbent core 51 to the rear end edge of the rear waistline region S2.

According to the present aspect, the difference between the distance from the folding line to the rear end edge of the absorbent core and the distance from the folding line to the front end edge of the absorbent core is longer than the second distance from the rear end edge of the absorbent core to the rear end edge of the rear waistline region, and hence the relatively high rigidity absorbent core extends long rearward the rear waistline region. In this case, when grasping the vicinity of the side engaging portion to pull up the absorbent article to the wearer's waistline, the pulling force is sufficiently transmitted to the absorbent core arranged in the rear waistline region. This makes it easy to pull up the absorbent core beyond the swelling of the wearer's buttocks.

For example, in an S size disposable diaper an embodiment, the first distance L1 from the front end edge of the absorbent core 51 to the front end edge of the front waistline region S1 is longer than the second distance L2 from the rear end edge of the absorbent core 51 to the rear end edge of the rear waistline region S2. The second distance L2 from the rear end edge of the absorbent core 51 to the rear end edge of the rear waistline region S2 is longer than the length L4 in the front-rear direction L of the region of the front waistline region S1 overlapping the absorbent core 51. Furthermore, a difference L3 between the distance from the folding line FL to the rear end edge of the absorbent core 51 and the distance from the folding line FL to the front end edge of the absorbent core 51 is longer than a length L4 in the front-rear direction L of the region overlapping the absorbent core 51 of the front waistline region S1. Furthermore, the difference L3 between the distance from the folding line FL to the rear end edge of the absorbent core 51 and the distance from the folding line FL to the front end edge of the absorbent core 51 is longer than the second distance L2 from the rear end edge of the absorbent core 51 to the rear end edge of the rear waistline region S2.

The liquid-impermeable film constituting the non-skin surface sheet 42 of the absorbent body 40 may extend to the front side relative to the absorbent core 51 toward the front side in the front-rear direction L. The region on the front side relative to the front end edge of the liquid-impermeable film constituting the non-skin surface sheet 42 accounts for preferably 50% or more, more preferably 60% or more, of the length of the side engaging portion 60 in the front-rear direction L. That is, the region of preferably 50% or more, more preferably 60% or more of the front waistline region S1 does not have a liquid-impermeable film. According to the present aspect, since the region without a liquid-impermeable film more solid than a sheet such as a nonwoven fabric is generally widened, it is possible to secure a wide region suitable for wiping the wearer's skin.

Preferably, an entire front region S5 of the front waistline region S1 on the front side relative to the front end edge of the absorbent core 51 is constituted of four or more sheets. That is, the entire front region S5 includes four or more layers of sheets stacked on one another. In the state where the rear waistline region S2 is laid under the wearer's buttocks and the side engaging portion 60 is torn, the entire front region S5 corresponds to a region where the wearer's skin is easily wiped. The entire region where the wearer's skin is easily wiped is constituted of four or more layers of sheets, and the body fluid hardly oozes and the assistant's hands hardly get dirty.

The number of sheets constituting the entire front region S5 is preferably equal to or less than the number of sheets constituting the rear region (region corresponding to the second distance L2 in Fig. 4) of the rear waistline region S2 overlapping the front region S5 in the state of being folded at the folding line FL. It is possible to suppress the rigidity of the front region from becoming too high by not excessively increasing the number of sheets of the front region S5, and it is possible to easily wipe off the body fluid adhering to the skin by the front region S5. On the other hand, by not excessively reducing the number of sheets in the rear region (region corresponding to the second distance L2 in Fig. 4), when grasping the vicinity of the side engaging portion 60 to pull up the absorbent article 10 to the wearer's waistline, the pulling force becomes easy to be sufficiently transmitted to the absorbent core 51.

The basis weight of the sheet constituting the entire front region S5 is preferably higher than the basis weight of the sheet constituting the rear region (region corresponding to the second distance L2 in Fig. 4) of the rear waistline region S2 overlapping the front region in the state of being folded at the folding line FL. By increasing the basis weight of the sheet in the front region S5, it is possible to make it easy to sufficiently wipe the body fluid adhering to the skin by the front region S5.

Preferably, an elastic thread-free region 80 (region hatched in Fig. 2) without elastic threads accounts for a continuous region of at least 30%, more preferably at least 50%, of the entire front waistline region S1. In the region having the elastic thread, there is a difference in contraction force between the part provided with the elastic thread and the part around it. The difference in the contraction force causes a large unevenness in the sheet between the elastic threads. In the present aspect, since the elastic thread-free region 80 without elastic threads accounts for a wide region, a large unevenness accompanying the contraction force of the elastic thread does not occur in a relatively wide range in the front waistline region S1. The region where such large unevenness is not generated is a region suitable for wiping the wearer's skin.

At least a part of the elastic thread-free region 80 is preferably located on the front side relative to the center of the side engaging portion 60 in the front-rear direction L. According to the present aspect, the elastic thread-free region 80 suitable for wiping the wearer's skin is located on the front side (ventral side). In the state where the rear waistline region S2 is laid under the wearer's buttocks and the side engaging portion 60 is torn, the operation range of the region on the front side (ventral side) of the front waistline region becomes relatively wide. Accordingly, it becomes easy for the assistant to wipe the wearer's skin using the elastic thread-free region 80 in the state where the side engaging portion 60 is torn.

The elastic thread-free region 80 preferably extends rearward from the front end edge of the front waistline region S1. In the state where the rear waistline region S2 is laid under the wearer's buttocks and the side engaging portion 60 is torn, the operation range of the region on the front side (ventral side) of the front waistline region S1 becomes relatively wide. Accordingly, since the elastic thread-free region 80 extends rearward from the front end edge of the front waistline region S1, it is easier for the assistant to wipe the wearer's skin using the elastic thread-free region 80 in the state where the side engaging portion 60 is torn.

At least a part of the elastic thread-free region 80 is provided in a region where the absorbent core 51 is extended forward in the front-rear direction L. The region where the absorbent core 51 is extended forward is close to the region where the wearer's skin gets dirty by the body fluid adhered to the absorbent core 51. Accordingly, by providing the elastic thread-free region 80 suitable for wiping the wearer's skin in the region where the absorbent core 51 is extended forward, it becomes easy to wipe the dirt adhered to the wearer's skin in the elastic thread-free region 80.

At least a part of the elastic thread-free region 80 is preferably provided outside the absorbent core 51 in the width direction W. The region of the front waistline region S1 outside the absorbent core 51 in the width direction W is a region hardly gets dirty by the body fluid of the wearer. By providing the elastic thread-free region 80 suitable for wiping the wearer's skin in such a stain-resistant region, it becomes possible to wipe the dirty adhered to the wearer's skin using a clean part of the front waistline region.

It is more preferable that the elastic thread-free region 80 extends from the center in the width direction W to the outside of the absorbent core 51 in the width direction W. In this case, since the elastic thread-free region 80 is widened in the width direction, it becomes possible to wipe the dirt adhering to the wearer's skin using a part of the elastic thread-free region 80 according to the preference of the assistant.

The elastic thread-free region 80 preferably has the stretchable sheet 90 stretchable in the width direction W. By providing the elastic thread-free region 80 in the front waistline region S1 with the stretchable sheet 90 stretchable in the width direction W, the front waistline region S1 can be made stretchable in the width direction W. Due to this, it is possible to make the front waistline region S1 easy to fit to the wearer's skin.

Furthermore, the stretchable sheet 90 tends to contract uniformly in the range where the stretchable sheet 90 exists. Therefore, a large unevenness occurring with the contraction of the elastic thread is hardly generated. Accordingly, the elastic thread-free region 80 in the front waistline region S1 can be fitted to the wearer's skin by the stretchable sheet 90, and the elastic thread-free region 80 can be maintained as a region suitable for wiping the wearer's skin.

It is preferable that the elastic thread-free region 80 does not have a liquid-impermeable film. That is, the front waistline member 20 in the elastic thread-free region 80 does not have a liquid-impermeable film, and may be constituted of a liquid-permeable nonwoven fabric, for example. Furthermore, while the non-skin surface sheet 42 of the absorbent body 40 has a liquid-impermeable film as described above, it is preferable that the liquid-impermeable film does not reach the elastic thread-free region 80. In a case where a liquid-impermeable film more solid than a sheet such as a nonwoven fabric is not provided in the elastic thread-free region 80, the elastic thread-free region 80 can be made more suitable for wiping the wearer's skin.

Instead of the above-described configuration, the elastic thread-free region 80 may have a liquid-impermeable film. For example, the liquid-impermeable film constituting the non-skin surface sheet 42 of the absorbent body 40 may reach the elastic thread-free region 80. In the case where the liquid-impermeable film is present in the elastic thread-free region 80 suitable for wiping the skin, when the assistant wipes the body fluid adhering to the wearer's skin in the elastic thread-free region 80, the body fluid adhering to the elastic thread-free region 80 can be suppressed from passing through, in the thickness direction, the sheet constituting the elastic thread-free region 80 and adhering to the assistant's hands.

In the present embodiment, the numerical values relating to the "length" of each part of the absorbent article 10 are measured in the stretched state of the absorbent article 10 stretched until no wrinkles (excluding the folding line FL) are present in the absorbent article 10 when the absorbent article 10 is developed by releasing the side engaging portion 60.

Although the embodiment of the present invention has been described above in detail, it is apparent for persons who have ordinary skill in the art that the embodiment described in this specification do not limit the scope of the present invention. Changes and modifications may apply to the embodiment of the present invention without departing from the scope of the present invention that are defined by the description of the appended claims. The description of the present specification, therefore, has been understood to be illustrative and is in no way intended to limit the scope of the invention.

For example, in the above embodiment, the absorbent body 40 is configured separately from the front waistline member 20 and the rear waistline member 30. Alternatively, the absorbent body 40 may be configured integrally with the front waistline member 20 and the rear waistline member 30.

In the above embodiment, the absorbent article 10 is a so-called underpants type absorbent article having the side engaging portion 60. Alternatively, the absorbent article 10 may be a tape type absorbent article. In this case, the absorbent article 10 does not have the side engaging portion 60, and may have a fastening tape that detachably fixes the front waistline region S1 and the rear waistline region S2 to each other.

### [INDUSTRIAL APPLICABILITY]

It is possible to provide an absorbent article easy to wear when putting on and easy to wipe the wearer's skin when removing.

### [REFERENCE SIGNS LIST]

- 10: Absorbent article
- 40: Absorbent body
- 50: Absorber
- 51: Absorbent core
- 60: Side engaging portion
- 80: Elastic thread-free region
- S1: Front waistline region
- S2: Rear waistline region
- S3: Crotch region
- FL: Folding line
- L: Front-rear direction
- W: Width direction
- T: Thickness direction

## Claims

1. An absorbent article (10), comprising:
a front-rear direction (L);
a width direction (W) orthogonal to the front-rear direction;
a front waistline region (S1) ;
a rear waistline region (S2) ;
a crotch region (S3) arranged between the front waistline region and the rear waistline region;
a side engaging portion (60) engaging the front waistline region and the rear waistline region; and
an absorbent core (51) provided at least in the crotch region, wherein
a folding line (FL) for folding the absorbent article in half extends along the width direction in the crotch region,
a first distance (L1) from a front end edge of the absorbent core (51) to a front end edge of the front waistline region (S1) is longer than a second distance (L2) from a rear end edge of the absorbent core (51) to a rear end edge of the rear waistline region (S2), and
a difference (L3) between a distance from the folding line (FL) to a rear end edge of the absorbent core (51) and a distance from the folding line (FL) to a front end edge of the absorbent core (51) is longer than a length (L4) in the front-rear direction of a region of the front waistline region (S1) overlapping the absorbent core (51), and
wherein the difference (L3) between a distance from the folding line (FL) to a rear end edge of the absorbent core (51) and a distance from the folding line (FL) to a front end edge of the absorbent core (51) is longer than the second distance (L2).

2. The absorbent article according to claim 1, further comprising:
an absorbent body (40) having the absorbent core (51) and a liquid-impermeable film provided on a non-skin surface side (T2) of the absorbent core, wherein
a region on a front side relative to a front end edge of the liquid-impermeable film accounts for 50% or more of a length of the side engaging portion in the front-rear direction.

3. The absorbent article according to claim 1 or 2, wherein an entire front region of the front waistline region (S1) on a front side relative to a front end edge of the absorbent core (51) is constituted by four or more sheets.

4. The absorbent article according to any one of claims 1 to 3, wherein a number of sheets constituting an entire front region (S5) of the front waistline region (S1) on a front side relative to a front end edge of the absorbent core (51) is equal to or less than a number of sheets constituting a rear region of the rear waistline region (S2) overlapping the front region in a state of being folded at the folding line (FL).

5. The absorbent article according to any one of claims 1 to 4, wherein a basis weight of a sheet constituting an entire front region (S5) of the front waistline region (S1) on a front side relative to a front end edge of the absorbent core (51) is higher than a basis weight of a sheet constituting a rear region of the rear waistline region (S2) overlapping the front region in a state of being folded at the folding line (FL).

6. The absorbent article according to any one of claims 1 to 5, wherein an elastic thread-free region (80) not having an elastic thread accounts for a continuous region of at least 30% of the entire front waistline region.

7. The absorbent article according to claim 6, wherein the elastic thread-free region (80) has a stretchable sheet (90) stretchable in the width direction.

8. The absorbent article according to claim 6 or 7, wherein at least a part of the elastic thread-free region (80) is located on a front side relative to a center of the side engaging portion in the front-rear direction.

9. The absorbent article according to any one of claims 6 to 8, wherein the elastic thread-free region (80) extends rearward from a front end edge of the front waistline region (S1).

10. The absorbent article according to any one of claims 6 to 9, wherein at least a part of the elastic thread-free region (80) is provided in a region where the absorbent core (51) is extended in the front-rear direction.

11. The absorbent article according to any one of claims 6 to 10, wherein at least a part of the elastic thread-free region (80) is provided outside the absorbent core (51) in the width direction.

12. The absorbent article according to any one of claims 6 to 11, wherein the elastic thread-free region (80) does not have a liquid-impermeable film.

13. The absorbent article according to any one of claims 6 to 11, wherein the elastic thread-free region (80) has a liquid-impermeable film.

## Patentansprüche

1. Absorbierender Artikel (10), der Folgendes umfasst:
eine Vorn-Hinten-Richtung (L);
eine Breitenrichtung (W) rechtwinklig zu der Vorn-Hinten-Richtung;
einen vorderen Taillenbereich (S1);
einen hinteren Taillenbereich (S2);
einen Schrittbereich (S3), der zwischen dem vorderen Taillenbereich und dem hinteren Taillenbereich angeordnet ist;
einen Seiteneingriffsteil (60), der in den vorderen Taillenbereich und den hinteren Taillenbereich eingreift; und
einen Saugkern (51), der zumindest in dem Schrittbereich bereitgestellt ist, wobei:
sich eine Faltlinie (FL) zum Falten des absorbierenden Artikels zur Hälfte entlang der Breitenrichtung in dem Schrittbereich erstreckt,
eine erste Entfernung (L1) von einem vorderen Endrand des Saugkerns (51) zu einem vorderen Endrand des vorderen Taillenbereichs (S1) länger ist als eine zweite Entfernung (L2) von einem hinteren Endrand des Saugkerns (51) zu einem hinteren Endrand des hinteren Taillenbereichs (S2) und ein Unterschied (L3) zwischen einer Entfernung von der Faltlinie (FL) zu einem hinteren Endrand des Saugkerns (51) und
einer Entfernung von der Faltlinie (FL) zu einem vorderen Endrand des Saugkerns (51) länger ist als eine Länge (L4) in der Vorn-Hinten-Richtung eines Bereichs des vorderen Taillenbereichs (S1), der den Saugkern (51) überlappt, und
wobei der Unterschied (L3) zwischen einer Entfernung von der Faltlinie (FL) zu einem hinteren Endrand des Saugkerns (51) und einer Entfernung von der Faltlinie (FL) zu einem vorderen Endrand des Saugkerns (51) länger ist als die zweite Entfernung (L2).

2. Absorbierender Artikel nach Anspruch 1, der weiter Folgendes umfasst:
einen Saugkörper (40), der den Saugkern (51) und einen flüssigkeitsundurchlässigen Film, der auf einer Nicht-Hautoberflächenseite (T2) des Saugkerns bereitgestellt ist, aufweist, wobei
ein Bereich auf einer vorderen Seite in Bezug auf einen vorderen Endrand des flüssigkeitsundurchlässigen Films 50 % oder mehr einer Länge des Seiteneingriffsteils in der Vorn-Hinten-Richtung ausmacht.

3. Absorbierender Artikel nach Anspruch 1 oder 2, wobei ein gesamter vorderer Bereich des vorderen Taillenbereichs (S1) auf einer vorderen Seite in Bezug auf einen vorderen Endrand des Saugkerns (51) aus vier oder mehr Lagen gebildet ist.

4. Absorbierender Artikel nach einem der Ansprüche 1 bis 3, wobei eine Anzahl von Lagen, die einen gesamten vorderen Bereich (S5) des vorderen Taillenbereichs (S1) auf einer vorderen Seite in Bezug auf einen vorderen Endrand des Saugkerns (51) bilden, gleich oder kleiner ist als eine Anzahl von Lagen, die einen hinteren Bereich des hinteren Taillenbereichs (S2) bilden, der den vorderen Bereich in einem Zustand der Faltung an der Faltlinie (FL) überlappt.

5. Absorbierender Artikel nach einem der Ansprüche 1 bis 4, wobei ein Flächengewicht einer Lage, die einen gesamten vorderen Bereich (S5) des vorderen Taillenbereichs (S1) auf einer vorderen Seite in Bezug auf einen vorderen Endrand des Saugkerns (51) bildet, höher ist als ein Flächengewicht einer Lage, die einen hinteren Bereich des hinteren Taillenbereichs (S2) bildet, der den vorderen Bereich in einem Zustand der Faltung an der Faltlinie (FL) überlappt.

6. Absorbierender Artikel nach einem der Ansprüche 1 bis 5, wobei ein Bereich ohne elastischen Faden (80), der keinen elastischen Faden aufweist, einen kontinuierlichen Bereich von mindestens 30 % des gesamten vorderen Taillenbereichs ausmacht.

7. Absorbierender Artikel nach Anspruch 6, wobei der Bereich ohne elastischen Faden (80) eine dehnbare Lage (90) aufweist, die in der Breitenrichtung dehnbar ist.

8. Absorbierender Artikel nach Anspruch 6 oder 7, wobei sich zumindest ein Teil des Bereichs ohne elastischen Faden (80) auf einer vorderen Seite in Bezug auf eine Mitte des Seiteneingriffsteils in der Vorn-Hinten-Richtung befindet.

9. Absorbierender Artikel nach einem der Ansprüche 6 bis 8, wobei sich der Bereich ohne elastischen Faden (80) nach hinten von einem vorderen Endrand des vorderen Taillenbereichs (S1) erstreckt.

10. Absorbierender Artikel nach einem der Ansprüche 6 bis 9, wobei zumindest ein Teil des Bereichs ohne elastischen Faden (80) in einem Bereich bereitgestellt ist, wo sich der Saugkern (51) in der Vorn-Hinten-Richtung erstreckt.

11. Absorbierender Artikel nach einem der Ansprüche 6 bis 10, wobei zumindest ein Teil des Bereichs ohne elastischen Faden (80) außerhalb des Saugkerns (51) in der Breitenrichtung bereitgestellt ist.

12. Absorbierender Artikel nach einem der Ansprüche 6 bis 11, wobei der Bereich ohne elastischen Faden (80) keinen flüssigkeitsundurchlässigen Film aufweist.

13. Absorbierender Artikel nach einem der Ansprüche 6 bis 11, wobei der Bereich ohne elastischen Faden (80) einen flüssigkeitsundurchlässigen Film aufweist.

## Revendications

1. Article absorbant (10), comportant :
une direction allant d'avant en arrière (L) ;
une direction allant dans le sens de la largeur (W) qui est orthogonale par rapport à la direction allant d'avant en arrière ;
une région avant au niveau de la taille (S1) ;
une région arrière au niveau de la taille (S2) ;
une région au niveau de l'entrejambe (S3) agencée entre la région avant au niveau de la taille et la région arrière au niveau de la taille ;
une partie de mise en prise latérale (60) mettant en prise la région avant au niveau de la taille et la région arrière au niveau de la taille ; et
une partie centrale absorbante (51) mise en oeuvre au moins dans la région au niveau de l'entrejambe, dans lequel une ligne de pliage (FL) servant à plier l'article absorbant en deux s'étend le long d'une direction allant dans le sens de la largeur dans la région au niveau de l'entrejambe,
une première distance (L1) allant depuis un bord d'extrémité avant de la partie centrale absorbante (51) jusqu'à un bord d'extrémité avant de la région avant au niveau de la taille (S1) est plus longue qu'une deuxième distance (L2) allant depuis un bord d'extrémité arrière de la partie centrale absorbante (51) jusqu'à un bord d'extrémité arrière de la région arrière au niveau de la taille (S2), et une différence (L3) entre une distance allant depuis la ligne de pliage (FL) jusqu'à un bord d'extrémité arrière de la partie centrale absorbante (51) et une distance allant depuis la ligne de pliage (FL) jusqu'à un bord d'extrémité avant de la partie centrale absorbante (51) est plus longue qu'une longueur (L4) dans la direction allant d'avant en arrière d'une région de la région avant au niveau de la taille (S1) chevauchant la partie centrale absorbante (51), et
dans lequel la différence (L3) entre une distance allant depuis la ligne de pliage (FL) jusqu'à un bord d'extrémité arrière de la partie centrale absorbante (51) et une distance allant depuis la ligne de pliage (FL) jusqu'à un bord d'extrémité avant de la partie centrale absorbante (51) est plus longue que la deuxième distance (L2).

2. Article absorbant selon la revendication 1, comportant par ailleurs :
un corps absorbant (40) ayant la partie centrale absorbante (51) et un film imperméable aux liquides mis en oeuvre sur un côté de surface non en contact avec la peau (T2) de la partie centrale absorbante, dans lequel
une région sur un côté avant par rapport à un bord d'extrémité avant du film imperméable aux liquides représente 50 % ou plus d'une longueur de la partie de mise en prise latérale dans la direction allant d'avant en arrière.

3. Article absorbant selon la revendication 1 ou la revendication 2, dans lequel toute une région avant de la région avant au niveau de la taille (S1) sur un côté avant par rapport à un bord d'extrémité avant de la partie centrale absorbante (51) est constituée de quatre feuilles ou plus.

4. Article absorbant selon l'une quelconque des revendications 1 à 3, dans lequel un nombre de feuilles constituant toute une région avant (S5) de la région avant au niveau de la taille (S1) sur un côté avant par rapport à un bord d'extrémité avant de la partie centrale absorbante (51) est égal ou inférieur à un nombre de feuilles constituant une région arrière de la région arrière au niveau de la taille (S2) chevauchant la région avant dans un état qui est plié au niveau de la ligne de pliage (FL).

5. Article absorbant selon l'une quelconque des revendications 1 à 4, dans lequel une masse surfacique d'une feuille constituant toute une région avant (S5) de la région avant au niveau de la taille (S1) sur un côté avant par rapport à un bord d'extrémité avant de la partie centrale absorbante (51) est supérieure à une masse surfacique d'une feuille constituant une région arrière de la région arrière au niveau de la taille (S2) chevauchant la région avant dans un état qui est plié au niveau de la ligne de pliage (FL).

6. Article absorbant selon l'une quelconque des revendications 1 à 5, dans lequel une région exempte de fil élastique (80) qui n'a pas de fil élastique représente une région continue d'au moins 30 % de toute la région avant au niveau de la taille.

7. Article absorbant selon la revendication 6, dans lequel la région exempte de fil élastique (80) a une feuille extensible (90) qui est en mesure de s'étendre dans la direction allant dans le sens de la largeur.

8. Article absorbant selon la revendication 6 ou la revendication 7, dans lequel au moins une partie de la région exempte de fil élastique (80) est située sur un côté avant par rapport à un centre de la partie de mise en prise latérale dans la direction allant d'avant en arrière.

9. Article absorbant selon l'une quelconque des revendications 6 à 8, dans lequel la région exempte de fil élastique (80) s'étend vers l'arrière depuis un bord d'extrémité avant de la région avant au niveau de la taille (S1).

10. Article absorbant selon l'une quelconque des revendications 6 à 9, dans lequel au moins une partie de la région exempte de fil élastique (80) est mise en oeuvre dans une région où la partie centrale absorbante (51) est étendue dans la direction allant d'avant en arrière.

11. Article absorbant selon l'une quelconque des revendications 6 à 10, dans lequel au moins une partie de la région exempte de fil élastique (80) est mise en oeuvre à l'extérieur de la partie centrale absorbante (51) dans la direction allant dans le sens de la largeur.

12. Article absorbant selon l'une quelconque des revendications 6 à 11, dans lequel la région exempte de fil élastique (80) n'a pas de film imperméable aux liquides.

13. Article absorbant selon l'une quelconque des revendications 6 à 11, dans lequel la région exempte de fil élastique (80) a un film imperméable aux liquides.
